# EUROPEAN PATENT APPLICATION

(11) **EP 4 283 555 A1**
(43) Date of publication of application: **29.11.2023**
(21) Application number: 22753032.6
(22) Date of filing: 15.02.2022
(51) Int. Cl.: G06Q 50/12, G06Q 50/22, G16H 20/60, G06Q 30/06

(54) **MENU PROVIDING APPARATUS AND METHOD**

(30) Priority: 15.02.2021 KR 20210019695
(71) Applicant: Nuvi Labs Co., Ltd., Seoul 06210 (KR)
(72) Inventor: KIM, Dae Hoon, Gunpo-si, Gyeonggi-do 15851 (KR)
(74) Representative: dompatent von Kreisler Selting Werner - Partnerschaft von Patent- und Rechtsanwälten mbB
(86) International application number: PCT/KR2022/002218
(87) International publication number: WO 2022/173272

(57) **Abstract**

The present invention relates to a menu providing apparatus. The menu providing apparatus may comprise: a menu data collection unit which collects menu data through a network and stores the menu data in a menu database; a menu generation unit which generates menus by learning the menu data; a menu recommendation unit which transmits recommended menus to a user terminal on the basis of the generated menus and receives, from the user terminal, information about a selected menu and the number of meals; a menu modification unit which receives, from the user terminal, a modification command for the recommended menus, modifies the recommended menus according to the modification command, and transmits the same to the user terminal; and a food ingredient ordering unit which derives types of food ingredients according to the selected menu, calculates the required quantity for each type of food ingredient according to the number
of meals, and transmits the same to a food ingredient supplier terminal.

## Description

### [TECHNICAL FIELD]

Embodiments of the inventive concept described herein relate to an artificial intelligence (AI)-based menu providing apparatus and a menu providing method.

### [BACKGROUND ART]

Nowadays, catering services operated by kindergartens, daycare centers, schools, and corporate cafeterias usually have a single dietitian who composes daily menus, prepares food ingredients depending on the composed menus, and serves meals to children, students, or employees.

Moreover, dietitians order food ingredients according to a menu individually from food ingredient suppliers and receive the food ingredients at a cost.

### [DETAILED DESCRIPTION OF THE INVENTION]

### [TECHINICAL PROBLEM]

In a case of a service in which a conventional dietitian composes a menu and provides meals, the dietitian composes a daily menu, and preparing food ingredients according to the composed menu is a great stress for the dietitian. Accordingly, there is an increasing need for a technology capable of reducing the burden of dietitian's menu configuration and preparing food ingredients easily.

Furthermore, when a dietitian prepares a menu individually and orders food ingredients according to the prepared menu directly from ingredient suppliers, the food ingredients may be expensive as food ingredient suppliers needs to supply the food ingredients in small quantities. There is an increasing need for a technology capable of saving costs of food ingredients by unifying menus of many dietitians and supplying food ingredients in large quantities.

Problems to be solved by the inventive concept are not limited to the problems mentioned above, and other problems not mentioned will be clearly understood by those skilled in the art from the following description.

### [TECHNICAL SOLUTION]

According to an embodiment, this specification presents a menu providing apparatus. The menu providing apparatus includes a menu data collection unit that collects menu data over a network and to store the menu data in a menu database, a menu generation unit that generates a menu by learning the menu data, a menu recommendation unit that transmits a recommended menu to a user terminal based on the generated menu and to receive, from the user terminal, information about a selected menu and the number of meals, a menu modification unit that receives, from the user terminal, a modification command for the recommended menu, modifies the recommended menu depending on the modification command, and transmits the modified menu to the user terminal, and a food ingredient ordering unit that derives a type of a food ingredient according to the selected menu, calculates a required quantity for each type of a food ingredient according to the number of meals, and transmits the calculated result to a food ingredient supplier terminal.

The menu providing apparatus and other embodiments may include the following features.

According to an embodiment, the menu recommendation unit may compose the recommended menu based on a menu configuration condition input through the user terminal. The menu configuration condition may include at least one or more of a calorie of food that constitutes a menu, nutrient, taste, sweetness, a degree of spiciness, a combination of foods, a seasonal food ingredient, the number of side dishes, price of a food ingredient, event food, food in subdivisions of seasons, seasonal food, a meal target, redundancy of individual food that constitutes a menu, the number of meals per serving, preference, the number of menus at a food service center, an amount of leftover food, meal price, a color combination of foods, allergy information about a food ingredient, region information, and a menu evaluation result of another user.

According to an embodiment, moreover, the menu recommendation unit may send a recommended menu to the user terminal based on a statistical result of a menu configuration thus accumulated.

According to an embodiment, furthermore, the menu recommendation unit may composes the recommended menu based on food ingredient inventory information possessed by a food ingredient supplier from the food ingredient supplier terminal, food ingredient information thus mainly supplied, food ingredient supply and demand status information, and the menu configuration condition.

According to an embodiment, also, the food ingredient supply and demand status information may be determined based on food ingredient supply and demand fluctuation information including production of food ingredients, predicted production of food ingredients, export/import volume of food ingredients, and a change in demand for food ingredients.

According to an embodiment, the menu providing apparatus may further include a menu recording unit that records a menu composed by a user. The menu generation unit may learn a menu configuration pattern of the user based on the menu composed by the user, and may generate a menu based on a result obtained by learning the menu data and a learning result of the menu configuration pattern.

According to an embodiment, moreover, the communication unit may receive feedback on the recommended menu from the user terminal. The menu data collection unit may update the menu database based on the feedback. The menu generation unit may generate a menu based on the updated menu database and the feedback and may update a model that generates a menu based on the feedback.

According to an embodiment, furthermore, the food ingredient ordering unit may receive a quote for a food ingredient purchase for a quantity required for each type of the food ingredient from the food ingredient supplier terminal, may provide the quote to the user terminal, and may transmit an order for the food ingredient purchase to the food ingredient supplier terminal when receiving an approval for the quote for the food ingredient purchase from the user terminal.

According to an embodiment, also, the menu recommendation unit may group the user terminal by region based on a location of the food ingredient supplier and may recommend the same menu to a user terminal belonging to the same group. The food ingredient ordering unit may derive a type of a food ingredient corresponding to the same menu and the number of meals according to the same menu, may calculate a required quantity for the respective derived type of a food ingredient, and may send the calculated result to a food ingredient supplier terminal assigned to the same group.

According to an embodiment, moreover, the menu recommendation unit may generate an image of the selected menu based on information about the selected menu received from the user terminal, and may transmit the generated image of the menu to the user terminal.

According to an embodiment, furthermore, the food ingredient ordering unit may predict the number of meals for the selected menu, may calculate a required quantity of a food ingredient based on the predicted number of meals, and may send the calculated result to the food ingredient supplier terminal.

According to an embodiment, this specification presents a menu providing method of a menu providing apparatus recommending a menu by including a menu data collection unit, a menu generation unit, a menu recommendation unit, a menu modification unit, and a food ingredient ordering unit. The menu providing method includes collecting, by the menu data collection unit, menu data over a network, generating, by the menu generation unit, a menu by learning the menu data, transmitting, by the menu recommendation unit, a recommended menu to a user terminal based on the generated menu, receiving, from the user terminal, selected menu information or modified menu information about the recommended menu and information on the number of meals, and deriving, by the food ingredient ordering unit, a type of a food ingredient according to the selected menu information or the modified menu information, calculating a required quantity for each type of a food ingredient according to the number of meals, and transmitting the calculated result to a food ingredient supplier terminal.

The menu providing method and other embodiments may include the following features.

According to an embodiment, the menu recommendation unit may compose the recommended menu based on a menu configuration condition of a user input through the user terminal. The menu configuration condition may include at least one or more of a calorie of food that constitutes a menu, nutrient composition, taste, sweetness, a degree of spiciness, a combination of foods, a seasonal food ingredient, the number of side dishes, price of a food ingredient, event food, food in subdivisions of seasons, seasonal food, a meal target, redundancy of individual food that constitutes a menu, the number of meals per serving, preference, the number of menus at a food service center, an amount of leftover food, meal price, a color combination of foods, allergy information about a food ingredient, region information, a menu evaluation result of another user, and food ingredient supply and demand status information. The food ingredient supply and demand status information may be determined based on food ingredient supply and demand fluctuation information including production of food ingredients, predicted production of food ingredients, export/import volume of food ingredients, and a change in demand for food ingredients.

According to an embodiment, moreover, the menu recommendation unit may send a recommended menu to the user terminal based on a statistical result of a menu configuration thus accumulated.

According to an embodiment, furthermore, the menu providing apparatus may further include a menu recording unit that records a menu composed by a user. The menu generation unit may learn a menu configuration pattern of the user based on the menu composed by the user, and may generate a recommended menu based on a result obtained by learning the menu data and a learning result of the menu configuration pattern.

### [ADVANTAGEOUS EFFECTS OF THE INVENTION]

According to embodiments disclosed in this specification, a user's difficulty in preparing a menu may be removed by providing an optimal menu to the user.

Moreover, according to embodiments disclosed in this specification, because it is possible to provide the user with a menu recommended by AI according to the user's menu configuration condition, the user may easily prepare the menu.

Furthermore, according to embodiments disclosed in this specification, the user may receive food ingredients at a low price by providing a common menu to a plurality of users and allowing them to jointly purchase different food ingredients in a common menu. In addition, food ingredient suppliers may increase sales by supplying a large amount of food ingredients.

In the meantime, effects obtained in the inventive concept are not limited to the above-mentioned effects, and other effects that are not mentioned will be clearly understood by those skilled in the art from the following description.

### [DESCRIPTION OF THE DRAWINGS]

The following drawings attached to this specification illustrate preferred embodiments of the inventive concept, and serves to further understand the technical idea of the inventive concept together with the specific details for carrying out the inventive concept. Accordingly, the inventive concept should not be construed as being limited only to the matters described in such drawings.
FIG. 1 shows a perceptron structure.
FIG. 2 shows a structure of a multi-layer perceptron.
FIG. 3 shows an example of a deep neural network.
FIG. 4 shows an example of a convolutional neural network.
FIG. 5 shows a filter operation in a convolutional neural network.
FIG. 6 shows a neural network structure in which a recurrent loop is present.
FIG. 7 shows an operating structure of RNN.
FIG. 8 is a block diagram of an AI device, according to an embodiment of the inventive concept.
FIG. 9 shows a menu providing system, according to an embodiment of the inventive concept.
FIG. 10 is a block diagram showing a configuration of a menu providing apparatus, according to an embodiment of the inventive concept.
FIG. 11 shows an example for providing a menu and food ingredients by grouping users based on a region.
FIG. 12 shows an example of a table illustrating a part of menu data open to the public.
FIG. 13 shows an example of an image for a created menu.
FIG. 14 shows an example of an output screen providing detailed information on a recommended menu.
FIG. 15 shows an example of a user interface for modifying a menu for creating a customized menu.
FIG. 16 shows another example of a user interface for modifying a menu for creating a customized menu.
FIG. 17 illustrates a flow of data between components of a menu providing system, according to an embodiment of the inventive concept.

### [BEST MODE]

A technology disclosed in this specification may be applied to providing a menu for a meal service. However, the technology disclosed in this specification is not limited thereto, but may also be applied to all devices and methods to which the technical spirit of the technology may be applied.

It should be noted that technical terms used in this specification are only used to describe specific embodiments and are not intended to limit the spirit of the technology disclosed in this specification. Moreover, unless specifically defined otherwise in this specification, technical terms used in this specification should be interpreted in terms commonly understood by those of ordinary skill in the field to which the technology disclosed in this specification belongs, and should not be interpreted in an excessively comprehensive meaning or an excessively reduced meaning. Furthermore, when the technical terms used in this specification are incorrect technical terms that do not accurately express the spirit of the technology disclosed in this specification, it should be understood as being replaced with technical terms capable of being correctly understood by those skilled in the art in the field to which the technology disclosed in this specification belongs. Besides, general terms used in this specification should be interpreted as defined in advance or according to context, and should not be interpreted in an excessively reduced meaning.

Terms including ordinal numbers such as first and second used in this specification may be used to describe various components, but the components should not be limited by the terms. The terms are only used to distinguish one component from another component. For example, without departing the scope of the inventive concept, a first component may be referred to as a second component, and similarly, a second component may be referred to as a first component.

Hereinafter, the embodiments disclosed in this specification will be described in detail with reference to the accompanying drawings, but the same or similar components are assigned the same reference numerals regardless of reference numerals, and redundant description thereof will be omitted.

Moreover, in describing the scope and spirit of the inventive concept, when it is determined that the specific description of the known related art unnecessarily obscures the gist of the inventive concept, the detailed description thereof will be omitted. Furthermore, it should be noted that the accompanying drawings are only intended to facilitate understanding of the spirit of the technology disclosed in this specification, and should not be construed as limiting the spirit of the technology by the accompanying drawings.

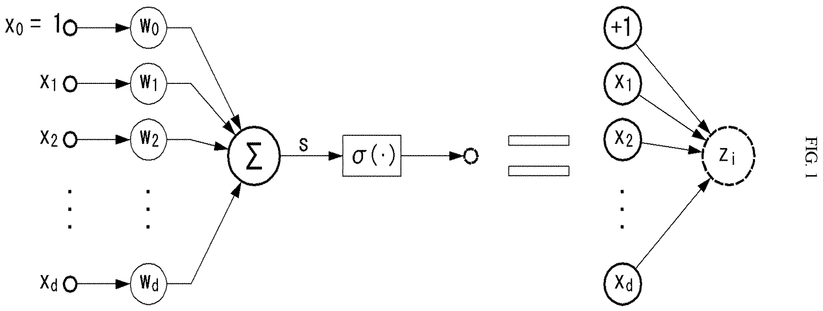

Hereinafter, artificial intelligence learning capable of being applied to a menu generation and menu recommendation function of a menu providing apparatus according to an embodiment of the inventive concept will be described with reference to accompanying FIGS. 1 to 8.

Artificial Intelligence (hereafter referred to as "AI") may determine a method of performing complex target tasks by using numerous analyzes. In other words, the AI may increase efficiency and may reduce processing delays. Time-consuming tasks such as analyzing large amounts of data may be performed instantly by using AI.

Hereinafter, machine learning, which is a type of AI, will be described in more detail.

The machine learning refers to a set of operations of learning a machine for generating a machine capable of doing tasks that humans is capable of doing or tasks that are difficult for humans to do. Data and a learning model are required for the machine learning. In the machine learning, data learning methods may be largely classified into three types: supervised learning, unsupervised learning, and reinforcement learning.

Neural network learning is used to minimize errors in an output. The neural network learning refers to a process of repeatedly inputting learning data into a neural network, calculating an output of the neural network for the learning data and an error of a target, and updating a weight of each node of the neural network by performing backpropagation on the error of the neural network from an output layer of the neural network to an input layer to reduce the error.

The supervised learning may use learning data having labeled correct answers in learning data, and the unsupervised learning may not have labeled correct answers in the learning data. That is, for example, learning data in a case of the supervised learning related to data classification may be data having the labeled category in each learning data. An error may be calculated by inputting the labeled learning data into the neural network, and comparing an output (category) of the neural network with the label of the learning data. The calculated error may be back-propagated in a reverse direction (i.e., a direction from the output layer to the input layer) in the neural network, and a connection weight of each node of each layer of the neural network may be updated depending on the back-propagation. A change amount of the connection weight of each updated node may be determined depending on a learning rate. The neural network's computation of input data and backpropagation of errors may constitute an epoch. The learning rate may be applied differently depending on the number of iterations of the epoch of the neural network. For example, in the early stages of neural network learning, a high learning rate may be used to increase efficiency by enabling the neural network to quickly achieve a specific level of performance. In the late stage of learning, a low learning rate may be used to increase accuracy.

The learning method may be different depending on characteristics of data. For example, it is preferable to perform learning by using the supervised learning rather than the unsupervised learning or reinforcement learning when a communication system is used to accurately predict data transmitted by a transmitting end at a receiving end.

A learning model may correspond to a human brain, and the most basic linear model may be considered as the learning model. A paradigm of machine learning that uses a neural network structure of high complexity, such as an artificial neural network (ANN), as the learning model is referred to as "deep learning".

There are three main types of neural network cores used as learning methods: deep neural networks (DNNs), convolutional deep neural networks (CNNs), and recurrent neural networks (RNNs).

An artificial neural network is an example of connecting several perceptrons.

FIG. 1 shows a perceptron structure.

Referring to FIG. 1, given the input vector x=(x₁, x₂, ..., x_{d}), an entire process of multiplying each component by a weight (W₁, W₂, ..., W_{d}), summing up the results, and then applying activation function σ(·) is referred to as a "perceptron". The huge artificial neural network structure may extend the simplified perceptron structure shown in FIG. 1 and may apply input vectors to different multi-dimensional perceptrons. For convenience of description, an input value or an output value is referred to as a "node".

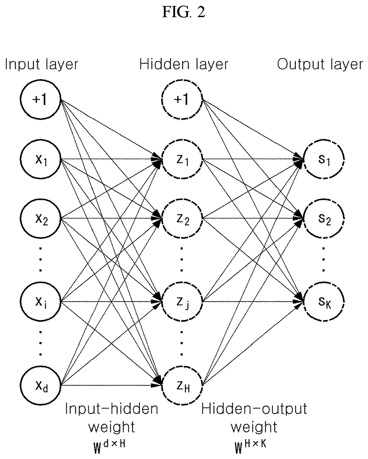

In the meantime, the perceptron structure shown in FIG. 1 may be described as consisting of a total of three layers based on input values and output values. An artificial neural network having H (d+1)-dimensional perceptrons between the first and second layers and K (H+1)-dimensional perceptrons between the second and third layers may be represented as shown in FIG. 2.

FIG. 2 shows a structure of a multi-layer perceptron.

A layer where an input vector is located is referred to as an "input layer"; a layer where a final output value is located is referred to as an "output layer"; and, all layers between the input and output layers are referred to as "hidden layers". FIG. 2 illustrates three layers. However, when the number of actual neural network layers is counted, the number of actual neural network layers is counted while the input layer is excluded, and thus the total number of layers may be considered as two layers. An artificial neural network is implemented by two-dimensionally connecting perceptrons of a basic block.

The input layer, hidden layer, and output layer described above may be applied in common in various artificial neural network structures, such as CNNs and RNNs, as well as multi-layer perceptrons. As the number of hidden layers increases, the ANN is deep. A machine learning paradigm that uses a sufficiently deep ANN as a learning model is called "deep learning". Moreover, the ANN used for deep learning is referred to as a "deep neural network (DNN)".

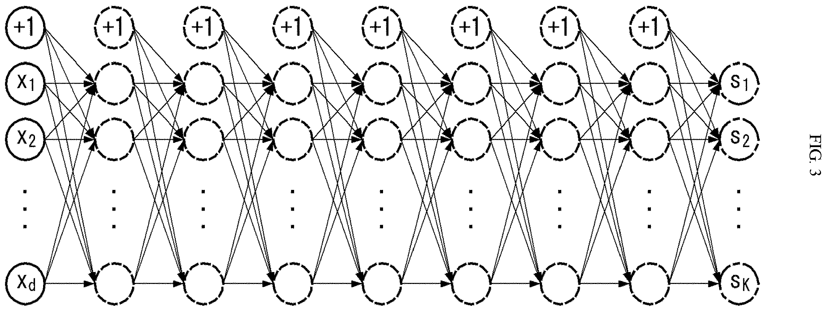

FIG. 3 shows an example of a deep neural network.

A deep neural network shown in FIG. 3 is a multi-layer perceptron having 8 layers (hidden layers + output layer). The multi-layer perceptron structure is expressed as a fully-connected neural network. In the fully-connected neural network, there is no connection relationship between nodes located on the same layer as each other, and the connection relationship is present only between nodes located on adjacent layers. The DNN may have a fully-connected neural network structure and may be composed of a combination of a plurality of hidden layers and activation functions. Accordingly, the DNN may be usefully applied to identify correlation characteristics between inputs and outputs. Here, the correlation characteristic may mean a joint probability of an input/output.

In the meantime, various artificial neural network structures different from the above-mentioned DNN may be formed based on how a plurality of perceptrons are connected to each other.

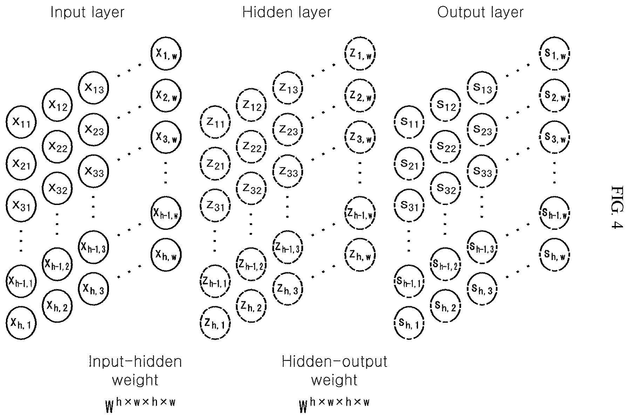

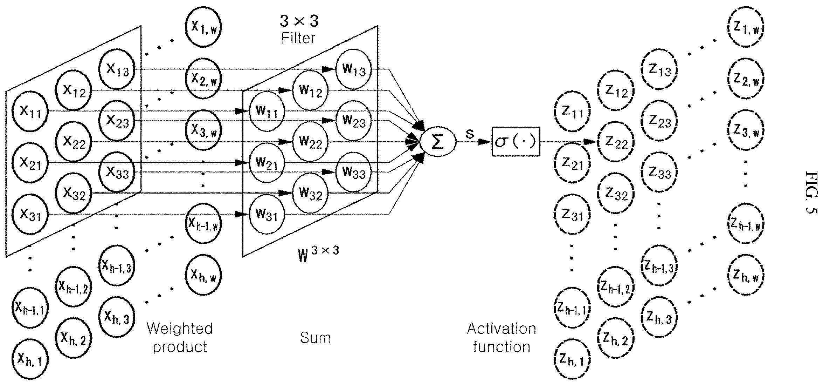

FIG. 4 shows an example of a convolutional neural network. FIG. 5 shows a filter operation in a convolutional neural network.

In DNN, nodes located inside one layer are positioned in a one-dimensional vertical direction. However, in FIG. 4, it may be assumed that the nodes are two-dimensionally arranged to have `w' horizontal nodes and 'h' vertical nodes (a convolutional neural network structure in FIG. 4). In this case, a weight is added for each connection in a process of connecting one input node to a hidden layer, and thus "h×w" weight needs to be considered. Because there are "h×w" nodes in an input layer, h²×w² weights are required between two adjacent layers.

In the convolutional neural network of FIG. 4, the number of weights may increase exponentially depending on the number of connections. It is assumed that a small-sized filter is present instead of considering the connection of all modes between adjacent layers. As shown in FIG. 5, it is necessary to perform a weighted sum and an activation function operation on the overlapping portion of filters.

One filter has a weight corresponding to the number corresponding to the size of the filter, and weights may be learned such that a specific feature on an image is capable of being extracted and output as a factor. In FIG. 5, a filter having a size of "3×3" is applied to the top left area having "3×3" on the input layer, and a result output value, which is obtained by performing the weighted sum and the activation function operation on the corresponding node, is stored in Z22.

While the input layer is scanned and a filter moves by a specific horizontal distance and a specific vertical distance, the weighted sum and the activation function operation are performed, and the output value is placed at a location of a current filter. This operation method is similar to a convolution operation for an image in a computer vision field, and thus the deep neural network of this structure is referred to as a "convolutional neural network (CNN)". A hidden layer generated as a result of the convolution operation is referred to as a "convolutional layer". In addition, a neural network having a plurality of convolutional layers is referred to as a "deep convolutional neural network (DCNN)".

The number of weights may be reduced by calculating a weighted sum by including only nodes located in an area covered by a filter in a node where the current filter is located in a convolution layer. For this reason, one filter may be used to focus on features for a local area. Accordingly, the CNN may be effectively applied to image data processing in which a physical distance in a two-dimensional area is an important criterion. In the meantime, in the CNN, a plurality of filters may be applied to a layer placed immediately before the convolution layer, and a plurality of output results may be generated through a convolution operation of each filter.

Meanwhile, there may be pieces of data in which sequence characteristics are important depending on data attributes. A structure in which a method of inputting one element on a data sequence at each timestep in consideration of the length variability of each of pieces of sequence data and a priority relationship between the pieces of sequence data, and inputting an output vector (a hidden vector) of a hidden layer output at a specific timestep together with the immediately next element on the sequence is applied to an ANN is referred to as an "RNN structure".

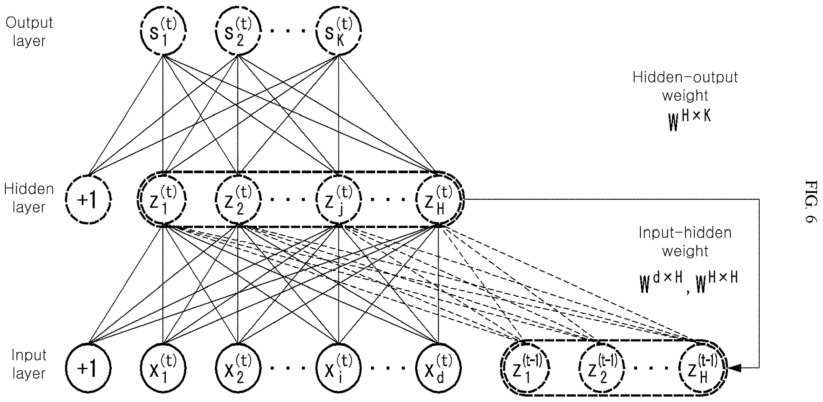

FIG. 6 shows a neural network structure in which a recurrent loop is present.

Referring to FIG. 6, a recurrent neural network (RNN) has a structure in which a weighted sum and an activation function are applied by inputting a hidden vector (z₁(t-1), z₂(t-1), ..., z_{H}(t-1)) at the immediately previous timestep `t-1' together in a process of inputting an element (x₁(t), x₂(t), ..., x_{d}(t)) at a timestep 't' on a data sequence to a fully-connected neural network. As such, the reason of delivering a hidden vector to the next timestep is that pieces of information in an input vector at the previous timesteps are considered to be accumulated in the hidden vector at a current timestep.

Referring to FIG. 6, the RNN operates in a predetermined timestep order for an input data sequence. Vector (z₁(2), z₂(2), ..., z_{H}(2)) of a hidden layer is determined through the weighted sum and activation function by entering hidden vector (z₁(1), z₂(1), ..., z_{H}(1)) at a point in time when input vector (x₁(t), x₂(t), ..., x_{d}(t)) at timestep 1 is input to the RNN together with input vector (x₁(2), x₂(2), ..., x_{d}(2)) at timestep 2. This process is repeated until timestep 2, timestep 3, ..., timestep T are arrived.

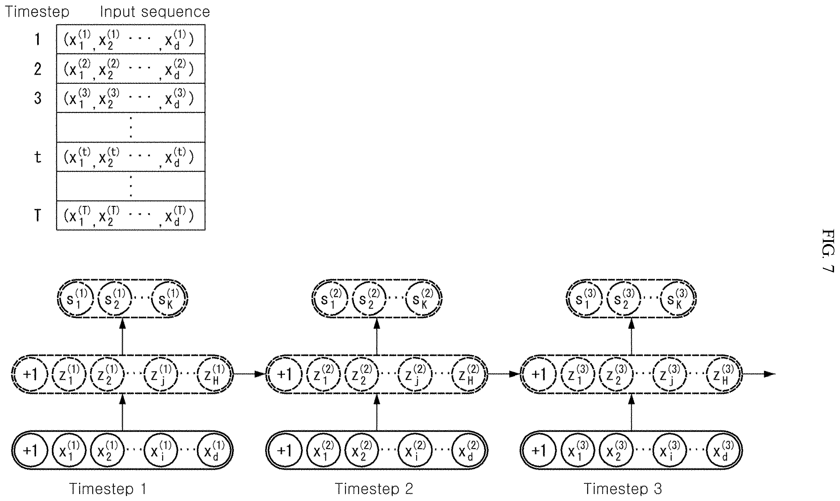

FIG. 7 shows an operating structure of RNN.

In the meantime, a case where a plurality of hidden layers are positioned in RNN is referred to as a "deep recurrent neural network (DRNN)". The RNN is designed to be usefully applied to sequence data (e.g., natural language processing).

The RNN may be a neural network core used as a learning method, and includes various deep learning techniques such as Restricted Boltzmann Machine (RBM), deep belief networks (DBN), and deep Q-network (Deep Q-Network) in addition to DNN, CNN, and RNN. The RNN may be applied to fields such as computer vision, speech recognition, natural language processing, voice/signal processing, and derivation of optimal values through large-capacity data processing.

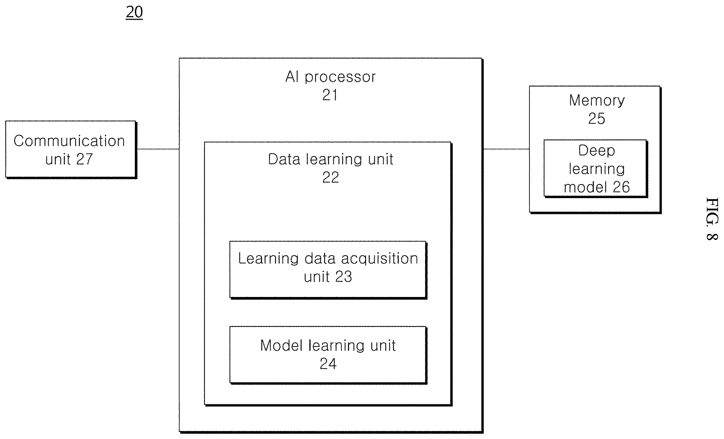

FIG. 8 is a block diagram of an AI device, according to an embodiment of the inventive concept.

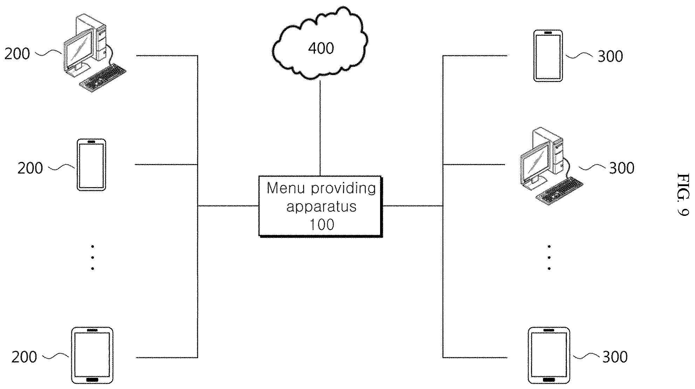

An AI device 20 may include an electronic device including an AI module capable of performing AI processing, a server including the AI module, or the like. In addition, the AI device 20 may be included in at least part of a menu providing apparatus 100 shown in FIG. 9 to perform at least part of AI processing together.

AI processing of the AI device 20 may include all operations related to control of the menu providing apparatus 100 shown in FIG. 9 and all operations for providing menus through artificial intelligence learning. For example, the menu providing apparatus 100 may perform an operation of processing/determining, generating a recommended menu, and recommending a menu by AI processing collected menu data. Moreover, for example, the menu providing apparatus 100 may generate menus and may recommend menus by AI-processing data obtained through interactions with other devices on a network, an open menu database, and other components provided in a user/food ingredient supplier terminal.

The AI device 20 may include an AI processor 21, a memory 25, and/or a communication unit 27.

The AI device 20 may be a computing device capable of learning a neural network, and may be implemented in various electronic devices such as a server, a desktop PC, a notebook PC, and a tablet PC. In the inventive concept, the AI device 20 may be a menu providing apparatus implemented in one form of various electronic devices.

The AI processor 21 may learn a neural network by using a program stored in the memory 25. In particular, the AI processor 21 may learn a neural network for recognizing device-related data. Here, the neural network for recognizing the device-related data may be designed to simulate a human brain structure on a computer, and may include a plurality of network nodes, each of which has a weight and which simulate neurons of a human neural network. The plurality of network modes may exchange data depending on each connection relationship such that neurons simulate synaptic activity of neurons that exchange signals through synapses. Here, the neural network may include a deep learning model developed from a neural network model. In the deep learning model, a plurality of network nodes may exchange data depending on a convolution connection relationship while being located on different layers. Examples of neural network models may include various deep learning techniques such as deep neural networks (DNN), convolutional deep neural networks (CNN), recurrent neural networks (RNN) restricted Boltzmann machine (RBM), deep belief networks (DBN), and deep Q-networks, and may be applied to fields such as computer vision, speech recognition, natural language processing, and speech/signal processing.

In the meantime, a processor performing functions described above may be a general-purpose processor (e.g., CPU), or may be an AI-dedicated processor (e.g., GPU) for artificial intelligence learning.

The memory 25 may store various programs and data, which are necessary for an operation of the AI device 20. The memory 25 may be implemented as a non-volatile memory, a volatile memory, a flash-memory, a hard disk drive (HDD), or a solid state drive (SSD). The memory 25 may be accessed by the AI processor 21, and data may be read/written/modified/deleted/updated by the AI processor 21. Furthermore, the memory 25 may store a neural network model (e.g., a deep learning model 26) generated through a learning algorithm for data classification/recognition according to an embodiment of the inventive concept.

In the meantime, the AI processor 21 may include a data learning unit 22 that learns a neural network for data classification/recognition. The data learning unit 22 may learn a criterion for learning data use to determine data classification/recognition, and a method of classifying and recognizing data by using the learning data. The data learning unit 22 may learn a deep learning model by obtaining learning data to be used for learning and applying the obtained learning data to a deep learning model.

The data learning unit 22 may be manufactured in a form of at least one hardware chip to be mounted on the AI device 20. For example, the data learning unit 22 may be manufactured in a type of a dedicated hardware chip for AI, and may be manufactured as a part of a general-purpose processor (CPU) or a graphic-dedicated processor (GPU) to be mounted on the AI device 20. Furthermore, the data learning unit 22 may be implemented as a software module. When the data learning unit 22 is implemented as a software module (or a program module including instructions), the software module may be stored in non-transitory computer readable media capable of being read by a computer. In this case, at least one software module may be provided by an operating system (OS) or an application (an application program).

The data learning unit 22 may include a learning data acquisition unit 23 and a model learning unit 24.

The learning data acquisition unit 23 may obtain learning data necessary for a neural network model for classifying and recognizing data. For example, the learning data acquisition unit 23 may obtain menu data and/or sample data, which is to be input to a neural network model, as learning data.

The model learning unit 24 may learn the neural network model such that the neural network model has a determination criterion for classifying predetermined data, by using the obtained learning data. In this case, the model learning unit 24 may learn the neural network model through supervised learning that uses at least some of the learning data as the determination criterion. Alternatively, the model learning unit 24 may learn the neural network model through unsupervised learning that discovers the determination criterion by learning by itself by using the learning data without supervision. Moreover, the model learning unit 24 may learn the neural network model through reinforcement learning by using feedback about whether the result of the situation determination according to learning is correct. Furthermore, the model learning unit 24 may learn a neural network model by using a learning algorithm including error back-propagation or gradient decent.

When the neural network model is learned, the model learning unit 24 may store the learned neural network model in a memory. The model learning unit 24 may store the learned neural network model in the memory of a server connected to the AI device 20 through a wired or wireless network.

The data learning unit 22 may further include a learning data pre-processing unit (not shown) and a learning data selection unit (not shown) to improve the analysis result of the recognition model or to save resources or time required for generating the recognition model.

The learning data pre-processing unit may pre-process the obtained data such that the obtained data is capable of being used for learning for situation determination. For example, the learning data pre-processing unit may process the obtained data in a predetermined format such that the model learning unit 24 is capable of using the obtained learning data for learning for menu data recognition.

Moreover, the learning data selection unit may select data necessary for learning from among learning data obtained by the learning data acquisition unit 23 or learning data preprocessed by the pre-processing unit. The selected learning data may be provided to the model learning unit 24. For example, the learning data selection unit may select only the data for a menu configuration included in a specific field as learning data by recognizing the specific field among the menu data collected through the network.

In addition, the data learning unit 22 may further include a model evaluation unit (not shown) to improve the analysis result of a neural network model.

The model evaluation unit may input evaluation data to the neural network model. When the analysis result output from the evaluation data does not satisfy a predetermined criterion, the model evaluation unit may allow the model learning unit 24 to learn again. In this case, the evaluation data may be predefined data for evaluating the recognition model. For example, when the number or ratio of the evaluation data having inaccurate analysis results exceeds a predetermined threshold from among the analysis results of the learned recognition model for the evaluation data, the model evaluation unit may evaluate that the evaluation data does not satisfy a predetermined criterion.

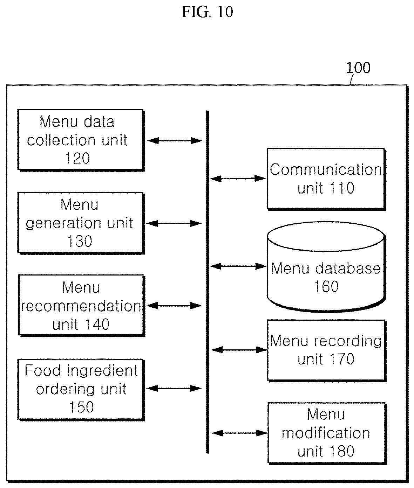

The communication unit 27 may transmit an AI processing result by the AI processor 21 to an external electronic device. Here, the external electronic device may be defined as a menu providing apparatus 100 (see FIG. 10). Meanwhile, the AI device 20 may be implemented by being operatively embedded in a menu generation unit 130 (see FIG. 10) included in a menu providing apparatus.

Meanwhile, it is described that the AI device 20 shown in FIG. 8 is functionally divided into the AI processor 21, the memory 25, the communication unit 27, or the like. However, it should be noted that the above-described components may be integrated into one module and then may be referred to as an "Al module".

Hereinafter, a method for providing a menu according to an embodiment of the inventive concept will be schematically described with reference to FIG. 9 attached thereto.

FIG. 9 shows a menu providing system, according to an embodiment of the inventive concept.

Referring to FIG. 9, a menu providing system disclosed herein may include the menu providing apparatus 100, one or more user terminals 200, and a food ingredient supplier terminal 300. The illustrated components are not essential, and thus a menu providing system having more or fewer components may be implemented. These components may be implemented in hardware or software, or through a combination of hardware and software.

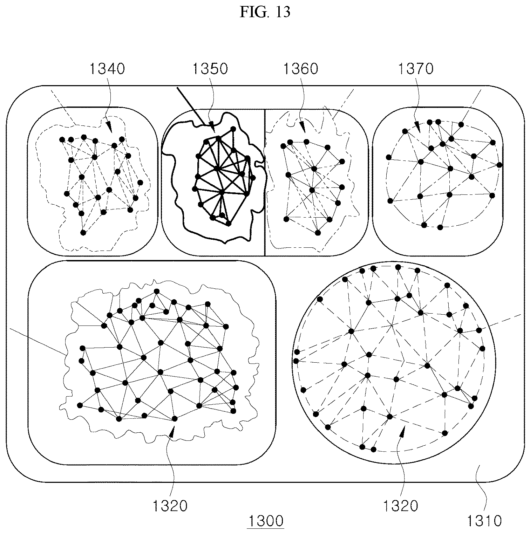

The menu providing apparatus 100 may collect menu data, which is open to the public, from a menu storage server 400 on a network and may store the menu data into a database. The public menu data may be menu data open to the public, such as menu data of daycare centers, kindergartens, elementary schools, middle schools, and high schools, and menu data of public hospitals and cafeterias of public institutions, and may be accessed and obtained by anyone through the network (see FIG. 13). Moreover, the menu providing apparatus 100 may collect menu data directly composed by a dietitian from users who have joined the system (i.e., the user terminal 200 of the dietitian), may store the menu data in a database.

The menu providing apparatus 100 may generate a menu by learning the collected menu data by using an AI-based menu configuration model and may transmit a recommended menu to the user terminal 200 based on the generated menu. In this case, the menu providing apparatus 100 may receive information about a menu selected by a user and the number of meals from the user terminal 200. Furthermore, the menu providing apparatus 100 may calculate the types of food ingredients and the amount of necessary food ingredients based on the received information about the number of meals and the menu, and may transmit, to the food ingredient supplier terminal 300, an order for food ingredients for each type of calculated ingredient.

Meanwhile, the above-described embodiment includes a process of generating a menu by using an AI model and then selecting a specific menu among recommended menus from the user terminal 200, but the present specification is not limited thereto. For example, the menu providing apparatus 100 may perform a process of generating a recommended menu based on a pre-learned menu recommendation model, transmitting the recommended menu to the user terminal 200, and calculating the amount of food ingredients required to be purchased through the generated recommended menu. In this case, a process of selecting a recommended menu and/or inputting information about the number of meals through the user terminal 200 may be omitted. The pre-registered meal number information may be used in a process of initially registering a service provided through the menu providing apparatus 100.

After identifying the menu data received from the menu providing apparatus 100, the user terminal 200 may input information about the number of meals for the recommended menu. In the meantime, a user (dietitian) may input requirements for his/her menu configuration and may receive a menu satisfying the requirements from the menu providing apparatus 100. For example, according to an embodiment of the present specification, the menu providing apparatus 100 may receive dietitian preference information from the user terminal 200 and may perform AI processing based on the input preference information and public data. The recommended menu generated as a result of the AI processing may be different for each school. In this case, a dietitian task may be reduced by automatically delivering menu recommendations and food ingredient orders to suppliers through AI processing.

The food ingredient supplier terminal 300 may receive a quote request or order for the purchase of food ingredients for a menu selected by a user (dietitian) from the menu providing apparatus 100. When an order is received after a quote is provided, a deal may be made to supply the corresponding food ingredients.

For example, the network disclosed in this specification may be a wireless network, a wired network, a public network such as Internet, a private network, a global system for mobile communication network (GSM) network, a general packet radio network (GPRN), a local area network (LAN), a wide area network (WAN), metropolitan area network (MAN), cellular network, public switched telephone network (PSTN), personal area network, Bluetooth, Wi-Fi Direct, near field communication, ultra-wide band, any combination thereof, or any other network, but is not limited thereto.

The menu providing apparatus 100, the one or more user terminals 200 and the food ingredient supplier terminal 300 in the menu providing system may be connected to one another through the above-mentioned network and may exchange data with each other.

Hereinafter, a configuration of a menu providing apparatus and a menu providing method according to an embodiment of the inventive concept will be described in detail with reference to FIGS. 10 to 17.

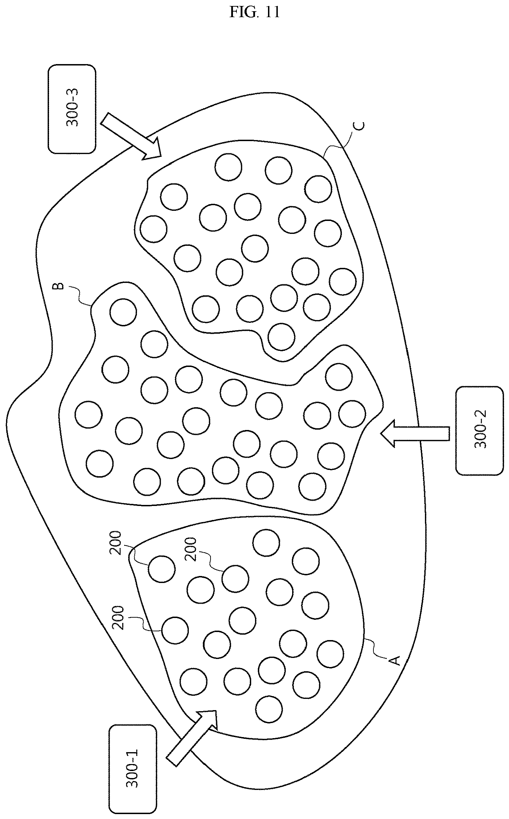

FIG. 10 is a block diagram showing a configuration of a menu providing apparatus, according to an embodiment of the inventive concept. FIG. 11 shows an example for providing a menu and food ingredients by grouping users based on a region.

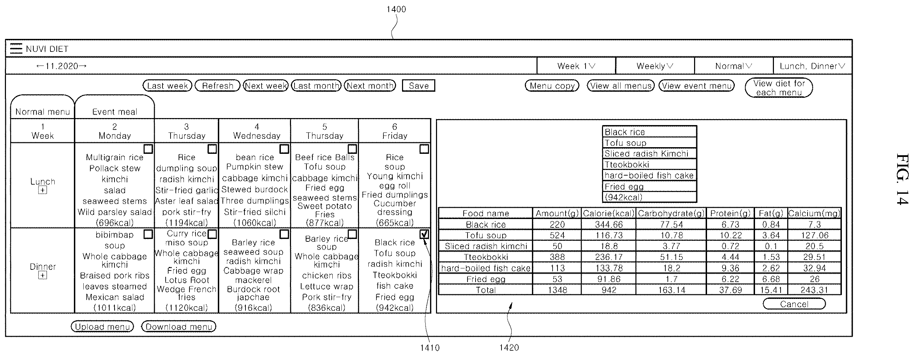

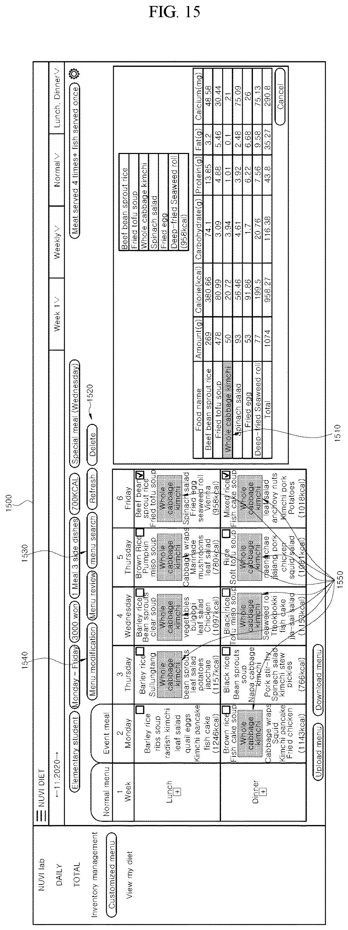

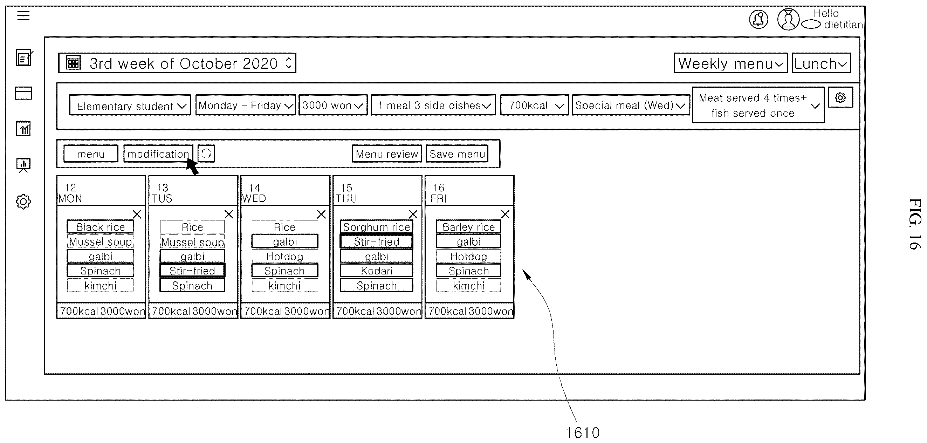

FIG. 13 shows an example of an image for a created menu. FIG. 14 shows an example of an output screen providing detailed information on a recommended menu. FIG. 15 shows an example of a user interface for modifying a menu for creating a customized menu. FIG. 16 shows another example of a user interface for modifying a menu for creating a customized menu.

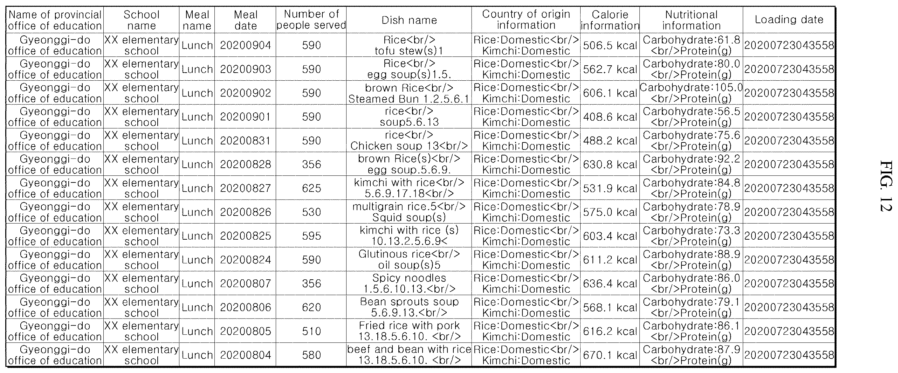

FIG. 12 shows a part of menu data open to the public in a table, which is capable of being downloaded and utilized by anyone from a public data portal provided by the government. A menu providing apparatus according to an embodiment of the inventive concept may create a menu optimized for a user's menu configuration condition or a menu capable of being used for general purposes by learning menu data collected from a portal.

Referring to FIG. 10, a menu providing apparatus 100 according to an embodiment of the inventive concept may include a communication unit 110, a menu data collection unit 120, a menu generation unit 130, a menu recommendation unit 140, a food ingredient ordering unit 150, a menu database 160, a menu recording unit 170, and a menu modification unit 180. The illustrated components are not essential, and thus a menu providing system having more or fewer components may be implemented. These components may be implemented in hardware or software, or through a combination of hardware and software.

The communication unit 110 may transmit and receive data by communicating with the user terminal 200 and the food ingredient supplier terminal 300 over a network.

The menu data collection unit 120 may collect menu data open to the public by connecting to a network through the communication unit 110 and may store the collected menu data in the menu database 160.

The menu generation unit 130 may create a menu by learning the collected menu data through an AI-based menu configuration model. The menu generation unit 130 may generate a standard menu or a general-purpose menu by processing the collected menu data through the AI menu configuration model. The menu generation unit 130 may generate a menu by inputting data such as public menu data shown in FIG. 12 into the AI menu configuration model. For example, the AI menu configuration model may receive and process data regarding a school, a meal date, the number of people served, a dish name (a food composition of a menu), country of origin information, calorie information, menu nutrition information, and unit price of food ingredients, and then may create a menu by date or meal target.

The menu recommendation unit 140 may transmit a recommended menu to the user terminal 200 based on the menu generated by the menu generation unit 130 and may receive information about a menu selected by a user (i.e., a dietitian) and the number of meals from the user terminal 200. The menu recommendation unit 140 may collectively provide the one or more connected user terminals 200 with standard menus or general-purpose menus, which are generated by the menu generation unit 130, depending on the characteristics of the food demander.

When receiving a confirmation message of the recommended menu from the user terminal 200, the menu recommendation unit 140 may generate an expected image for the corresponding menu. The predicted image may be synthesized by using an image database stored for each food or may be generated through a generative adversarial network (GAN). Accordingly, the user may effectively publicize a menu to be provided to meal recipients by using the menu image received from the menu providing apparatus 100. For example, as shown in FIG. 13, when receiving a confirmation message regarding menu selection from the user with respect to a menu consisting of brown rice, napa cabbage soybean paste soup, pickled garlic, vegetable salad, balloon flower root salad, and braised lotus root, the menu recommendation unit 140 may read images of confirmed foods from a pre-stored image database, may generate an image 1300 (i.e., an image for a menu selected by the user) obtained by synthesizing a plate image 1310 and a brown rice image 1320, napa cabbage soybean paste soup image 1330, a pickled garlic image 1340, a vegetable salad image 1350, a balloon flower root salad image 1360, and a braised lotus root image 1370, and may transmit the image 1300 to the user terminal 200.

Besides, the menu recommendation unit 140 may provide the user with nutrition information and calorie information for each menu with respect to the recommended menu. In addition, the menu recommendation unit 140 may provide the user with main color information for individual food for each menu with respect to the recommended menu. For example, as shown in FIG. 14, when a user selects a menu 1410 for dinner on November 6, 2020 in a state 1400 in which a menu provided by the menu providing apparatus 100 is output on the user terminal 200, the menu recommendation unit 140 may output a detailed screen 1420 for nutrition information, calorie information, and price information about the selected menu. Characters indicating a food name of individual food constituting the corresponding menu may be displayed on the detailed screen 1420 in the main color of the corresponding food. Moreover, nutritional components of the entire menu or nutritional components of the individual food may be displayed on the detailed screen 1420 in a predetermined color, and thus the menu may be modified by allowing the user to easily grasp the recommended menu based on the color information. Accordingly, after reviewing the output nutrition information, the output calorie information, and the output cost information, the user may modify the individual food to change nutrients, calories, and costs. The individual food for the recommended menu may be modified by the menu modification unit 180. The menu modification unit 180 may receive a modification command for the recommended menu displayed on the user's screen from the user and then may transmit the modified menu obtained by applying the modifications to the user terminal 200.

Moreover, the menu modification unit 180 of the menu providing apparatus 100 according to an embodiment of the inventive concept may receive a modification command for a recommended menu from the user terminal 200, may modify the recommended menu depending on the modification command, and may transmit the modified result to the user terminal 200 again. Furthermore, the menu modification unit 180 may provide a user interface 1500. The user may identify the number of times that specific food is provided during a specific period, with respect to a menu recommended by the menu recommendation unit 140 through the user interface 1500 and then may change the specific food to another food in consideration of the frequency of serving specific food The menu modification unit 180 may receive a modification command for the recommended menu input through the user interface 1500 and may modify the menu depending on the corresponding command. For example, as shown in FIG. 15, when a user wants to change "whole cabbage Kimchi" provided as a side dish to another side dish during the lunch menu on November 6, 2020, the user may select "whole cabbage Kimchi" in the user interface 1500 (1510), may delete "whole cabbage Kimchi" (1520), may search for a new side dish (1530), and may modify the menu to a new side dish thus found (1540). In this case, when the user selects "whole cabbage Kimchi", the user interface 1500 displays (1550) the name (i.e. "whole cabbage Kimchi") of the food selected from the output menu during a specific period in a specific color (1550) and outputs the name such that the user may easily identify a redundant provision status.

Moreover, as shown in FIG. 16, the user interface 1500 may display the same food in the same color in the menu of the specific period selected by the user (1610) such that the user perceives a redundant status of menus in the menu of a specific period. Accordingly, the user may adjust the menu so as to provide various menus by modifying redundant menus in the menu of a specific period output to the user interface 1500 through the menu modification unit 180.

The food ingredient ordering unit 150 may derive the types of food ingredients according to the menu selected by a dietitian, may calculate the required quantity for each type of a food ingredient according to the number of meals, and may transmit the counted result to the food ingredient supplier terminal 300.

Furthermore, the food ingredient ordering unit 150 may predict the number of meals for a specific meal or for a meal in a specific period, may calculate the required quantity for each type of a food ingredient depending on the predicted number of meals, and may automatically transmit the counted result to the food ingredient supplier terminal 300. The food ingredient ordering unit 150 may predict the number of meals based on leftover amount information, leftover information, weather information, information about the number of past meals, information about day of the week, menu information, event status information, time and attendance information/attendance information, and the like. When a food service center is a business, the food ingredient ordering unit 150 may predict the number of meals on a specific date or specific meal by utilizing a company's attendance information. When a food service center is an educational institution such as a school or kindergarten, the food ingredient ordering unit 150 may predict the number of meals for a specific date or specific meal by using the attendance information of the corresponding educational institution. For example, the food ingredient ordering unit 150 may predict demand for the corresponding menu based on past leftover information and/or leftover information for the recommended and selected menu or a menu modified by the user. In addition, the food ingredient ordering unit 150 may predict demand by utilizing weather information about a day when the corresponding menu is provided. For example, when the weather is predicted to rain on the day on which "noodle soup" is provided, the food ingredient ordering unit 150 may increase the number of supplied meals of noodle soup.

Besides, the food ingredient ordering unit 150 may receive a purchase quote for the quantity required for each type of a food ingredient from the food ingredient supplier terminal 300 and may provide the purchase quote to the user terminal 200. When receiving an approval of the food ingredient purchase quote from the user terminal 200, the food ingredient ordering unit 150 may transmit a purchase order for the food ingredients to the food ingredient supplier terminal 300. In this case, the user may review the purchase quote received from the food ingredient supplier terminal 300 and then may place a purchase order with the food ingredient supplier who offered the best price, thereby reducing the cost of purchasing food ingredients.

For example, when the menu recommendation unit 140 provides the same menu to the plurality of user terminals 200, the food ingredient supplier may receive bulk orders for a small number of food ingredients, which has benefits in terms of food ingredient inventory management, food ingredient delivery, and cost. The benefits may be a reason that the user pays a price to a menu providing service provider.

According to an embodiment, the menu recommendation unit 140 may compose a recommended menu based on the user's menu configuration condition input through the user terminal 200 and then may provide the composed menu to the user terminal 200.

The menu configuration condition may include at least one or more of the calories of food that constitutes a menu, nutrient composition, taste, sweetness, a degree of spiciness, a combination of foods, a seasonal food ingredient, the number of side dishes, price of a food ingredient, event food, food in subdivisions of seasons, seasonal food, a meal target (it may refer to a person receiving a meal and may be classified into elementary school students, middle school students, high school students, and the general public), redundancy of individual food that constitutes a menu, the number of meals per serving, preference, the number of menus at a food service center, an amount of leftover food, meal price, a color combination of foods, allergy information about a food ingredient, information of a region where a meal is served, and a menu evaluation result (an evaluation result of a dietitian using a menu providing apparatus elsewhere with respect to the generated menu) of another user. For example, when receiving, through the user terminal 200, an input for requesting a menu, of which the target is lower grade children in an elementary school, which has seasonal food ingredients used for food at the second of the three dog days, and which has the number of three side dishes, the menu recommendation unit 140 may compose a menu having a main menu including one jujube, one small ginseng root, brown rice with glutinous rice, and half of chicken soup with ginseng of 400 g, side dishes including cucumber pepper dressing and sliced radish kimchi, and a dessert including a piece of Korean melon among menu data generated by the menu generation unit 130 in consideration of the menu configuration condition, and may transmit the menu as a recommended menu to the user terminal 200. For example, when a user reviews the menu evaluation results of other users received through the user terminal 200, and then menu evaluations of other users having good results are reflected to the menu configuration condition under control of the user, the menu recommendation unit 140 may provide the menu to the user after composing a menu with foods that have good menu evaluation results by other users. A redundancy item of each food constituting the menu in the menu configuration condition may indicate the number of times that specific food is served during a specific period. When the redundancy of individual food constituting the menu is reflected to the menu configuration through the user terminal 200 under control of the user, the menu recommendation unit 140 may compose a menu such that the corresponding food is not repeatedly provided during a predetermined period. In addition, a long-term menu may be composed such that the overall statistical value of the menu configuration does not deviate. Menu evaluation results of other users may be collected through a user community provided by the menu providing apparatus 100. The user community may collect evaluation results evaluated by users with respect to menus that the users have selected or directly composed and shared, and may provide a user with the collected evaluation results as a selection menu of the menu configuration condition. The evaluation results may be classified by region and meal target, and may include various pieces of information such as the amount of orders for specific menus, the amount of leftovers, and the preference of a customer.

Moreover, in addition to creating recommended menus through learning of the collected menu data, the menu generation unit 130 may perform statistical analysis based on past menu data composed by the user and may utilize the analysis result to generate a recommended menu. The menu generation unit 130 may analyze the user's menu during a predetermined period, may add the statistical results for each dish to the menu configuration condition, and may create a menu based on the menu configuration condition including the statistical results. The menu generation unit 130 may derive statistics on food included in the corresponding element for each element of the menu configuration condition and may compose a menu such that the composition of the menu to be created later follows the derived result. For example, on the basis of a menu configuration condition, individual food such as bean sprout soup served 39 times, cabbage kimchi served 620 times, and stir-fried anchovies served 65 times may be analyzed based on statistics during a specific period, and a menu to be newly recommended may be composed such that the existing statistics are capable of being maintained. For example, when a menu including stir-fried anchovies served 3 times as a side dish, which is not spicy, was served last month, a menu may be composed this month such that stir-fried anchovies are provided three times as a side dish that is not spicy.

Also, according to an embodiment, an AI processor may use a plurality of menu generation models to create a menu. The plurality of menu generation models may be selected depending on preference information input through the user terminal 200. For example, the menu generation model may be composed of at least one learning model capable of providing different menus as output values depending on the age of a meal target. For example, a meal menu for elementary school students and a meal menu for high school students need to have different menu configurations in consideration of nutrients, calories, and the amount of food. Even when the main menu is the same, it is necessary to use different menu configuration models to compose additional menus and amounts of food ingredients differently.

Moreover, the type of the menu generation model may be a model that provides a menu composed for each season as an output value in addition to the age of the meal target. Furthermore, the menu generation model may be a model trained to output an optimal supplementary menu to the type of a main menu.

According to an embodiment, the menu recommendation unit 140 may compose a menu in consideration of inventory status information of food ingredients possessed by a food ingredient supplier from the food ingredient supplier terminal 300 or the type of food ingredients thus mainly supplied and may transmit a recommended menu to the user terminal 200. For example, the menu recommendation unit 140 may use a lot of stocked food ingredients based on the inventory status information of food ingredients received from the food ingredient supplier terminal 300 and may compose and provide a menu so as to use fewer food ingredients with low inventory. Besides, the menu recommendation unit 140 may automatically manage the inventory of food ingredients by reflecting other users' order quantity information about food ingredients to the inventory status of food ingredients in real time, thereby allowing a user to arbitrarily compose a menu according to the inventory status.

Also, the menu recommendation unit 140 may compose a recommended menu based on food ingredient supply and demand information and/or menu configuration conditions. The food ingredient supply and demand information may be determined based on food ingredient supply and demand fluctuation information including production of food ingredients, predicted production of food ingredients, export/import volume of food ingredients, and a change in demand for food ingredients. The menu recommendation unit 140 may obtain food ingredient supply and demand status information from a website of agricultural products distribution information (e.g., KAMIS agricultural distribution information) provided by public institutions such as the Korea Agro-Fisheries & Food Trade Corporation. The menu recommendation unit 140 may obtain the supply and demand situation and price information of food ingredients based on the agricultural products distribution information and may compose a menu with an optimal price based on the obtained information. In the meantime, the production forecast information of food ingredients may be predicted based on weather forecast information and/or past weather information. The menu recommendation unit 140 may predict the supply status of food ingredients based on weather information, and may compose a menu depending on the prediction result. For example, when a typhoon is predicted to severely damage specific agricultural products depending on the weather forecast, the menu recommendation unit 140 may create a menu using other substitutes without selecting a menu using the specific agricultural product at a point in time when the supply situation of the specific agricultural product deteriorates, thereby reducing the cost of menu configuration and contributing to price stabilization of agricultural products. Furthermore, for example, when the corresponding agricultural products are overproduced because the harvest of specific agricultural products is good, the menu recommendation unit 140 may provide users with a menu that uses the corresponding agricultural products as food ingredients, thereby contributing to stabilizing the market by promoting consumption of agricultural products as well as lowering the cost of school meals. In other words, the menu recommendation unit 140 may compose a menu based on the supply and demand status of food ingredients including a current supply status of food ingredients, a future supply forecast result, a demand status of food ingredients, a demand forecast result of food ingredients, or the like.

According to an embodiment, the communication unit 110 may receive feedback on the recommended menu from the user terminal 200. The menu data collection unit 120 may update a menu database based on the feedback, and the menu generation unit 130 may generate an improved menu by reflecting the updated menu database and feedback. Besides, the menu generation unit 130 may update the menu configuration model by learning an AI-based menu configuration model that generates a menu by reflecting feedback from the user terminal 200.

According to an embodiment, the menu recommendation unit 140 may group one or more users by region based on a location of the food ingredient supplier and may recommend the same menu to user terminals belonging to the same group. Also, the food ingredient ordering unit 150 may derive types of food ingredients corresponding to the same menu and the number of meals according to the same menu, may calculate the required quantity for each type of the derived food ingredient, and may transmit a food ingredient quote request and a food ingredient purchase order to a food ingredient supplier terminal assigned to the same group.

The menu recording unit 170 may record a menu composed by the user or another user, and may call a recording unit before using this device. After the menu generation unit 130 may learn a user's menu configuration pattern based on the user's configuration menu recorded in the menu recording unit 170, and then may create a menu based on the menu data learning result and the learning result of the menu configuration pattern. A user may save a menu, which he/she has composed, in a favorite through a favorite menu of the menu recording unit 170, may read out the favorites stored in the menu recording unit 170 as necessary, and may utilize the favorite when composing a new menu.

The menu generation unit 130 and the menu recommendation unit 140 may create standard menus and optimized menus by analyzing menu data through AI learning and AI processing shown in FIGS. 1 to 8, and may create and recommend a menu depending on the user's request.

For example, referring to FIG. 11, when food ingredient suppliers are located in three regions, the menu recommendation unit 140 may group the plurality of user terminals 200 into three groups A, B, and C in consideration of locations of the food ingredient suppliers, and then may match terminals of food ingredient suppliers located in close proximity to each group. Accordingly, the menu recommendation unit 140 may match a food ingredient supplier terminal 300-1 with the user terminal group (A), may match a food ingredient supplier terminal 300-2 with the user terminal group (B), and may match a food ingredient supplier terminal 300-3 with the user terminal group (C), thereby supplying food ingredients for the same menu between the matched user and the food ingredient supplier. In this way, the food ingredient supplier may save the time required for delivery of food ingredients, and may reduce costs by efficiently performing delivery. This cost reduction may lower the unit cost of supplying food ingredients, and thus users may obtain economic benefits.

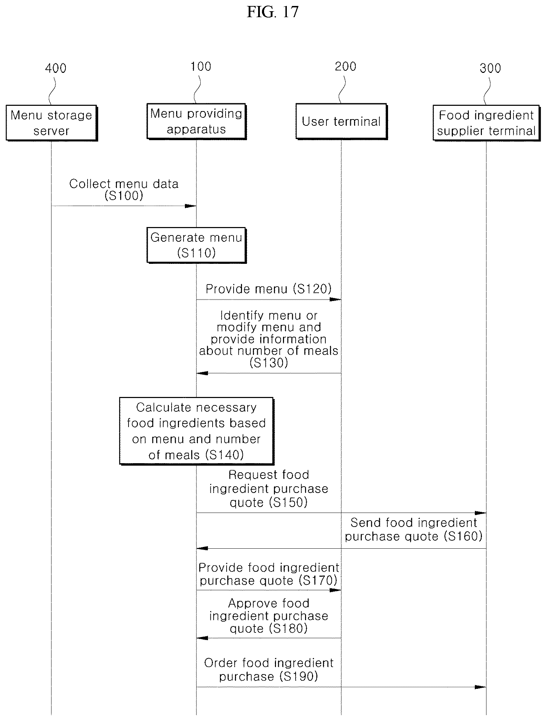

FIG. 17 illustrates a flow of data between components of a menu providing system, according to an embodiment of the inventive concept.

A menu providing method of the menu providing apparatus 100 recommending a menu by including the menu data collection unit 120, the menu generation unit 130, the menu recommendation unit 140, and the food ingredient ordering unit 150 will be described through the flow of data or signals between components of FIG. 17.

First of all, the menu data collection unit 120 may collect menu data open to the public through a network and may store the menu data in a database (S110).

Next, the menu generation unit 130 may generate a standard menu or common menu by learning the collected menu data (S120).

The menu recommendation unit 140 may transmit a recommended menu to the user terminal 200 based on the menu generated by the menu generation unit 130 and may receive information about the selected menu and the number of meals from the user terminal 200 (S130).

Next, the food ingredient ordering unit 150 may derive the types of food ingredients according to the selected menu, may calculate the required quantity for each type of the food ingredient according to the number of meals (S140), and may make a request for a quote for the purchase of food ingredients based on the calculated quantity of food ingredients to the food ingredient supplier terminal 300 (S150).

When receiving a request for a food ingredient purchase quote from the menu providing apparatus 100, the food ingredient supplier terminal 300 may make and reply with a quote for the requested food ingredients (S160). The menu providing apparatus 100 may provide the user terminal 200 with the quote received from the food ingredient supplier terminal 300.

When a user reviews and approves the quote (S180), the food ingredient ordering unit 150 of the menu providing apparatus 100 may order food ingredients by sending a food ingredient purchase order for the corresponding quote to the food ingredient supplier terminal 300 providing the corresponding quote (S190). In this case, because the user terminal 200 receives a plurality of food ingredient purchase quotes returned from the plurality of food ingredient supplier terminals 300 from the menu providing apparatus 100, the user may compare the plurality of quotes and may select the one that the user likes the most.

When the provision of menus and the supply of food ingredients are completed in a menu providing system, the menu providing apparatus 100 may charge service usage fees to the user terminal 200 and the food ingredient supplier terminal 300, and may act as an agent for payment for the supply of food ingredients between the user terminal 200 and the food ingredient supplier terminal 300.

In the above description, operation S100 to operation S190 may be further divided into additional operations or may be combined into fewer operations, according to an embodiment of the inventive concept. Moreover, some operations may be omitted as necessary, and the order between operations may be changed.

The term "unit/part" (e.g., a control unit/part) used herein may represent, for example, a unit including one or more combinations of hardware, software and firmware. For example, the term "unit/part" may be interchangeably used with the terms "unit", "logic", "logical block", "component" and "circuit". The "unit/part" may be a minimum unit of an integrated component or may be a part thereof. The "unit/part" may be a minimum unit for performing one or more functions or a part thereof. The "unit/part" may be implemented mechanically or electronically. For example, the "module" may include at least one of an application-specific IC (ASIC) chip, a field-programmable gate array (FPGA), and a programmable-logic device for performing some operations, which are known or will be developed.

At least a portion of an apparatus (e.g., modules or functions thereof) or a method (e.g., operations) according to various embodiments may be, for example, implemented by instructions stored in a computer-readable storage media in the form of a program module. The instruction, when executed by the processor, may cause the one or more processors to perform a function corresponding to the instruction. The computer-readable storage media, for example, may be a memory.

A computer-readable storage medium/computer-readable recording medium may include a hard disk, a magnetic media, a floppy disk, a magnetic media (e.g., a magnetic tape), an optical media (e.g., a compact disc read only memory (CD-ROM) and a digital versatile disc (DVD), a magneto-optical media (e.g., a floptical disk), and hardware devices (e.g., a read only memory (ROM), a random access memory (RAM), or a flash memory). Also, a program instruction may include not only a mechanical code such as things generated by a compiler but also a high-level language code executable on a computer using an interpreter. The above-mentioned hardware device may be configured to operate as one or more software modules to perform operations according to various embodiments of the inventive concept, and vice versa.

Modules or program modules according to various embodiments of the inventive concept may include at least one or more of the above-mentioned components, some of the above-mentioned components may be omitted, or other additional components may be further included therein. Operations executed by modules, program modules, or other elements may be executed by a successive method, a parallel method, a repeated method, or a heuristic method. Also, some of operations may be executed in different sequences, omitted, or other operations may be added.

As used herein, the term "one" is defined as one or is defined as one or more. Moreover, even though the same claim includes introductory phrases such as "at least one" and "one or more" and ambiguous phrases such as "one", the use of introductory phrases such as "at least one" and "one or more" in a claim should not be construed to mean that the introduction of another claim element by the ambiguous phrase "one" limits a specific claim that includes the claim element thus introduced to the inventive concept that includes only one such element.

Unless otherwise indicated, terms such as "first" and "second" are used to arbitrarily distinguish between elements described by such terms. Accordingly, these terms are not necessarily intended to indicate any temporal or other priority of such elements. The mere fact that certain means are recited in different claims does not indicate that a combination of such means is incapable of being used to be advantaged. Accordingly, these terms are not necessarily intended to indicate the temporal or other priority of such elements. The mere fact that certain measures are cited in different claims does not indicate that a combination of these measures is incapable of being useful.

An arrangement of components to achieve the same function is effectively "related" such that the desired function is achieved. Accordingly, two components combined to achieve a specific functionality may be considered to be "related" to each other in such a way that the desired functionality is achieved regardless of the structure or mediating component. Likewise, two components thus associated may be considered as being "operatively connected" or "operatively coupled" to each other to achieve a desired function.

Furthermore, those skilled in the art may perceive that a boundary between functionalities of the above-described operations is only an example. A plurality of operations may be combined into a single operation, a single operation may be distributed into additional operations, and the operations may be executed to at least partially overlap each other in time. Besides, alternative embodiments may include a plurality of instances of a specific operation, and the order of operations may vary in various other embodiments. However, other modifications, variations and alternatives are also possible. Accordingly, detailed descriptions and drawings need to be regarded in an illustrative rather than a limiting sense.

A phrase "may be X" indicates that condition X is capable of being satisfied. This phrase also indicates that condition X is incapable of being satisfied. For example, a reference to a system including a specific component needs to include scenarios where the system does not include the specific component. For example, a reference to a method that includes a specific operation needs to include scenarios where the corresponding method does not include the specific operation. However, for another example, a reference to a system configured to perform a specific operation should also include scenarios where the system is not configured to perform the specific operation.

The terms "comprising", "having", "consisting of", "composed of" and "consisting essentially of" are used interchangeably. For example, any method may include an operation included in at least drawings and/or specifications, and may include only the operation included in the drawings and/or specifications.

Those skilled in the art will recognize that a boundary between logic blocks is merely illustrative, and that alternative embodiments may merge logic blocks or circuit elements, or impose alternative decompositions of functionality on various logic blocks or circuit elements. Accordingly, it should be understood that the architecture shown herein is merely illustrative, and in fact many other architectures that achieve the same functionality is capable of being implemented.

Also, for example, in an embodiment, the illustrated examples may be implemented on a single integrated circuit or as a circuit located within the same device. Alternatively, the examples may be implemented as any number of discrete integrated circuits or discrete devices interconnected with one another in a suitable method, and other variations, modifications, variations and alternatives are also possible. Accordingly, the specification and drawings need to be regarded in an illustrative rather than a limiting sense.

Also, for example, the examples or portions thereof may be implemented as code representations or software of physical circuits or logical representations translatable to physical circuits, such as any suitable type of a hardware description language.

Moreover, the inventive concept is not limited to physical devices or units implemented with non-programmable hardware. However, the inventive concept may also be applied to a programmable device or unit, which is capable of performing desired device functions by operating in accordance with appropriate program code, such as a mainframes, a mini computer, a server, a workstation, a personal computer, a notepad, a personal digital information terminal (PDA), an electronic game, a vehicle, other embedded systems, a mobile phone, and various other wireless devices, and which is generally referred to herein as a 'computer system'.

A system, device or device described herein includes at least one hardware component.

For example, connections as described herein may be any type of connection suitable for transmitting a signal from a respective node, unit or device or to a respective node, unit or device via an intermediate device. Accordingly, unless implicitly or otherwise stated, a connection may be, for example, a direct connection or an indirect connection. The connection may be described or described with reference to a single connection, multiple connections, unidirectional connections, or bidirectional connections. However, different embodiments may change the implementation of a connection. For example, a separate unidirectional connection may be used instead of the bidirectional connection, and vice versa. Besides, the multiple connections may be replaced with a single connection for transmitting a plurality of signals sequentially or in a time multiplexed manner. Likewise, the single connection carrying a plurality of signals may be broken into various connections for transmitting subsets of these signals. Accordingly, there are many options for transmitting signals.

Those skilled in the art will recognize that a boundary between logic blocks is merely illustrative, and that alternative embodiments may merge logic blocks or circuit elements, or impose alternative decompositions of functionality on various logic blocks or circuit elements. Accordingly, it should be understood that the architecture shown herein is merely illustrative, and in fact many other architectures that achieve the same functionality is capable of being implemented.

In a claim, any reference numerals placed between parentheses shall not be construed as limiting the claim. A word 'comprising' does not exclude the presence of elements or acts recited in a claim.

In the above, a preferred embodiment of a technology of this specification has been described with reference to the accompanying drawings. Here, terms or words used in the present specification and claims should not be construed as being limited to ordinary or dictionary meanings, but should be interpreted as meanings and concepts consistent with the technical spirit of the inventive concept. The scope of the inventive concept is not limited to embodiments disclosed herein, and the inventive concept may be modified, changed, or improved in various forms within the spirit the inventive concept and the scope described in the claims.

## Claims

1. A menu providing apparatus comprising:
a menu data collection unit configured to collect menu data over a network and to store the menu data in a menu database;
a menu generation unit configured to generate a menu by learning the menu data;
a menu recommendation unit configured to transmit a recommended menu to a user terminal based on the generated menu and to receive, from the user terminal, information about a selected menu and the number of meals;
a menu modification unit configured to receive, from the user terminal, a modification command for the recommended menu, to modify the recommended menu depending on the modification command, and to transmit the modified menu to the user terminal; and
a food ingredient ordering unit configured to derive a type of a food ingredient according to the selected menu, to calculate a required quantity for each type of a food ingredient according to the number of meals, and to transmit the calculated result to a food ingredient supplier terminal.

2. The menu providing apparatus of claim 1, wherein the menu recommendation unit composes the recommended menu based on a menu configuration condition input through the user terminal, and
wherein the menu configuration condition includes at least one of a calorie of food that constitutes a menu, nutrient, taste, sweetness, a degree of spiciness, a combination of foods, a seasonal food ingredient, the number of side dishes, price of a food ingredient, event food, food in subdivisions of seasons, seasonal food, a meal target, redundancy of individual food that constitutes a menu, the number of meals per serving, preference, the number of menus at a food service center, an amount of leftover food, meal price, a color combination of foods, allergy information about a food ingredient, region information, and a menu evaluation result of another user.

3. The menu providing apparatus of claim 1, wherein the menu recommendation unit sends a recommended menu to the user terminal based on a statistical result of a menu configuration thus accumulated.

4. The menu providing apparatus of claim 2, wherein the menu recommendation unit composes the recommended menu based on food ingredient inventory information possessed by a food ingredient supplier from the food ingredient supplier terminal, food ingredient information thus mainly supplied, food ingredient supply and demand status information, and the menu configuration condition.

5. The menu providing apparatus of claim 4, wherein the food ingredient supply and demand status information is determined based on food ingredient supply and demand fluctuation information including production of food ingredients, predicted production of food ingredients, export/import volume of food ingredients, and a change in demand for food ingredients.

6. The menu providing apparatus of claim 1, further comprising:
a menu recording unit configured to record a menu composed by a user,
wherein the menu generation unit learns a menu configuration pattern of the user based on the menu composed by the user, and generates a menu based on a result obtained by learning the menu data and a learning result of the menu configuration pattern.

7. The menu providing apparatus of claim 1, wherein the communication unit receives feedback on the recommended menu from the user terminal,
wherein the menu data collection unit updates the menu database based on the feedback, and
wherein the menu generation unit generates a menu based on the updated menu database and the feedback and updates a model that generates a menu based on the feedback.

8. The menu providing apparatus of claim 1, wherein the food ingredient ordering unit receives a quote for a food ingredient purchase for a quantity required for each type of the food ingredient from the food ingredient supplier terminal, provides the quote to the user terminal, and transmits an order for the food ingredient purchase to the food ingredient supplier terminal when receiving an approval for the quote for the food ingredient purchase from the user terminal.

9. The menu providing apparatus of claim 1, wherein the menu recommendation unit groups the user terminal by region based on a location of the food ingredient supplier and recommends the same menu to a user terminal belonging to the same group, and
wherein the food ingredient ordering unit derives a type of a food ingredient corresponding to the same menu and the number of meals according to the same menu, calculates a required quantity for the respective derived type of a food ingredient, and sends the calculated result to a food ingredient supplier terminal assigned to the same group.

10. The menu providing apparatus of claim 1, wherein the menu recommendation unit generates an image of the selected menu based on information about the selected menu received from the user terminal, and transmits the generated image of the menu to the user terminal.

11. The menu providing apparatus of claim 1, wherein the food ingredient ordering unit predicts the number of meals for the selected menu, calculates a required quantity of a food ingredient based on the predicted number of meals, and sends the calculated result to the food ingredient supplier terminal.

12. A menu providing method of a menu providing apparatus recommending a menu by including a menu data collection unit, a menu generation unit, a menu recommendation unit, a menu modification unit, and a food ingredient ordering unit, the method comprising:
collecting, by the menu data collection unit, menu data over a network;
generating, by the menu generation unit, a menu by learning the menu data;
transmitting, by the menu recommendation unit, a recommended menu to a user terminal based on the generated menu;
receiving, from the user terminal, selected menu information or modified menu information about the recommended menu and information on the number of meals; and
deriving, by the food ingredient ordering unit, a type of a food ingredient according to the selected menu information or the modified menu information, calculating a required quantity for each type of a food ingredient according to the number of meals, and transmitting the calculated result to a food ingredient supplier terminal.

13. The method of claim 12, wherein the menu recommendation unit composes the recommended menu based on a menu configuration condition of a user input through the user terminal,
wherein the menu configuration condition includes at least one of a calorie of food that constitutes a menu, nutrient composition, taste, sweetness, a degree of spiciness, a combination of foods, a seasonal food ingredient, the number of side dishes, price of a food ingredient, event food, food in subdivisions of seasons, seasonal food, a meal target, redundancy of individual food that constitutes a menu, the number of meals per serving, preference, the number of menus at a food service center, an amount of leftover food, meal price, a color combination of foods, allergy information about a food ingredient, region information, a menu evaluation result of another user, and food ingredient supply and demand status information, and
wherein the food ingredient supply and demand status information is determined based on food ingredient supply and demand fluctuation information including production of food ingredients, predicted production of food ingredients, export/import volume of food ingredients, and a change in demand for food ingredients.

14. The method of claim 13, wherein the menu recommendation unit sends a recommended menu to the user terminal based on a statistical result of a menu configuration thus accumulated.

15. The method of claim 14, wherein the menu providing apparatus further includes a menu recording unit configured to record a menu composed by a user, and
wherein the menu generation unit learns a menu configuration pattern of the user based on the menu composed by the user, and generates a recommended menu based on a result obtained by learning the menu data and a learning result of the menu configuration pattern.
